# EUROPÄISCHE PATENTSCHRIFT

(11) **EP 0 893 465 B1**
(45) Veröffentlichungstag und Bekanntmachung des Hinweises auf die Patenterteilung: **06.10.2004**
(21) Anmeldenummer: 98112965.3
(22) Anmeldetag: 13.07.1998
(51) Int. Cl.: C08G 73/02, C09D 179/02, C09J 179/02

(54) **Selbsthärtende wässrige Bindemittel für flächige Beschichtungen, Verklebungen und Abdichtungen**
Self hardenining aqueous binders for coatings, adhesives and sealings
Liants aqueux autodurcissables pour revêtements, collages ou joints

(30) Priorität: 24.07.1997 DE 19731863
(43) Veröffentlichungstag der Anmeldung: 27.01.1999
(73) Patentinhaber: Bayer MaterialScience AG, 51368 Leverkusen (DE)
(72) Erfinder: Pedain, Josef Dr., 51061 Köln (DE); König, Klaus Dr., 51519 Odenthal (DE); Heitkämper, Peter Dr., 41542 Dormagen (DE); Schönfelder, Manfred, 51381 Leverkusen (DE)

(56) Entgegenhaltungen:
- EP-A- 0 548 930
- CHEMICAL ABSTRACTS, vol. 80, no. 16, 22. April 1974 Columbus, Ohio, US; abstract no. 83693, FUKUDA ET AL.: "Preparation of polymers by the reaction of bisoxadiazolones with diamines" XP002091418 & NIPPON KAGAKU KAISHI, Bd. 10, 1973, Seiten 1987-92,

## Beschreibung

Die vorliegende Erfindung betrifft sich in wäßriger Phase befindliche Kunststoffvorläufer, die bei erhöhter Temperatur und Verdunsten des Wassers zu vernetzten Kunststoffen aushärten.

In wäßriger Phase vorliegende Kunststoffe und auch Kunststoffverläufer sind bekannt. Genannt seien zum Beispiel wäßrige Polyurethan-Dispersionen oder wäßrige Polymerisat-Emulsionen und -Dispersionen. Sie lassen sich zu Kunststoffilmen auftrocknen und unter Umständen durch Zugabe von vernetzend wirkenden Härtern auch zu Wasser- und Lösemittel-beständigen Kunststoffkörpern aushärten. Nachteilig an allen bekannten wäßrigen Systemen dieser Art ist ihre begrenzte Verarbeitungszeit, wenn ein Vernetzer zugegeben wurde oder auch die Abspaltung von organischen Komponenten, die das Vernetzermolekül enthält. Aus blockierten Polyisocyanaten wird beispielsweise bei der Vernetzungsreaktion das Blockierungsmittel in Freiheit gesetzt. Aus veretherten Melaminformaldehydharzen, die ebenfalls großtechnisch eingesetzt werden, entsteht beim Aushärten u.a. ein Monoalkohol.

Sehr viele technisch verwendbare PUR-Dispersionen enthalten tert. Amine oder Ammoniak als Neutralisationsmittel, die beim Trocknen oder Aushärten den Film verlassen. Dabei können im Film nachteilige Poren entstehen sowie Geruchsbelästigungen für den Verarbeiter.

Aufgabe der Erfindung war es diese Nachteile zu vermeiden. Es wird ein vemetzbares, in Wasser gelöstes System aus Kunststoffvorläufern gefordert, das bei etwa 20 bis 30°C, also Umgebungstemperatur, mindestens beständig ist und bei erhöhter Temperatur unter Filmbildung vernetzt, ohne daß organische flüchtige Bestandteile den Film verlassen. Diese Aufgabe wurde mit Mischungen aus bestimmten Oxadiazolin-Derivaten und Polyaminen gelöst.

Gegenstand der Erfindung sind in wäßriger Phase vorliegende Mischungen aus Bis-(1.3.4-Oxadiazolin-5-onen) der Formel (I) 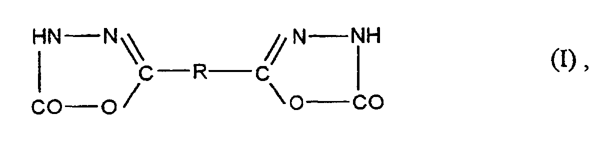 in der
- R: eine einfache Bindung oder einen bivalenten organischen Rest mit 1 bis 18 C-Atomen bedeutet, der aliphatisch, araliphatisch oder aromatisch sein kann,
mit organischen Polyaminen oder Polyaminabmischungen, die im Mittel mehr als 2 NH- und/oder NH₂-Gruppen enthalten.

Die Ausgangsprodukte der Formel (I) sind bekannt, ihre Herstellung, beispielsweise aus Carbonsäurehydraziden und Phosgen ist beschrieben. (Journal of the Chemical Society of Japan 1973, 10, 1987 - 1992). In der gleichen Literaturstelle werden auch Reaktionen der Substanzen (I) mit Diaminen beschrieben. Da die Substanzen (I) aber hochschmelzend und äußerst schwerlöslich sind, werden die Umsetzungen beispielsweise bei 160 bis 300°C in einem evakuierten Rohr bzw. im Stickstoffstrom durchgeführt.

Die Substanzen der Formel (I) lassen sich zusammen mit Polyaminen in Wasser auflösen und bei erhöhter Temperatur zur Reaktion bringen und zu sehr wertvollen Kunststoffen und Beschichtungen abreagieren. Dies ist sehr überraschend, weil die Verbindungen (I) in Wasser völlig unlöslich sind und auch die in Frage kommenden Polyamine ebenfalls in Wasser nicht oder nur wenig löslich sind. Ebenso ist überraschend, daß die wäßrigen Lösungen von Verbindungen der Formel (I) und Polyaminen z.B. bei 20 - 100°C und darüber filmbildend sind, obwohl die Substanzen niedermolekular und hochschmelzend sind.

Die Bis-oxadiazolinone der Formel (I) sind bekannt und werden auf bekannten Wegen hergestellt.

In der allgemeinen Formel (I) steht R vorzugsweise für den Rest (-CH₂)ₙ-, in welchem
- n: für eine Zahl von 0 bis 18 steht,
oder einem bifunktionellen aromatischen Rest der Formel (Ia),

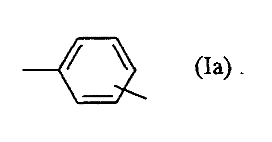

Die Herstellung eines typischen Vertreters der bevorzugt eingesetzt wird, 1,4-Bis-(1,3,4-oxadiazolin-5-on-2-yl)butan, beschreibt Beispiel 1.

Die in Frage kommenden Polyamine sind ebenfalls bekannt und in technischen Mengen zugänglich. Es kommen Triamine in Frage wie Diethylentriamin und Nonantriamin, aber auch Umsetzungsprodukte von Triolen mit Acrylnitril, die durch Hydrierung der Nitrilgruppen in bekannter Weise in Polyamine überführt werden. Gut geeignet sind auch Polyamine, die z.B. nach der EP 0 392 299 A hergestellt werden.

Die Polyamine mit einer Aminofunktionalität von 3 und größer können im allgemeinen mit Diaminen im Gemisch eingesetzt werden. Genannt seien Hexamethylendiamin, IPDA, 4,4'-Diamino-dicyclohexylmethan.

Bevorzugt finden Di- und Polyamine mit aliphatisch gebundenen Aminogruppen Verwendung.

Das Verhältnis von Verbindungen der Formel (I) zu den Polyaminen ist in einem breiten Bereich frei wählbar.

Für eine 1,3,4-Oxadiazolin-Gruppe können 0,5 bis 5 NH- bzw. NH₂-Gruppen Verwendung finden. Die Eigenschaften des ausgehärteten Kunststoff-Filmes lassen sich dadurch in gewissen Grenzen variieren.

Bevorzugt werden für eine Verbindung der Formel (I) 0,5 bis 10, besonders bevorzugt 2 bis 3 NH- bzw. NH₂-Gruppen eingesetzt.

Die wäßrigen Mischungen von Verbindungen der Formel (I) mit Polyaminen sind klare niedrigviskose Lösungen mit einem Feststoffgehalt von 20 - 70 %. Sie sind farblos oder hellgelb gefärbt und sind bis ca. 40°C beliebig lange beständig. Bei ihrem Auftrag auf ein Substrat wie beispielsweise Glas trocknen sie oberhalb von 50°C zu klaren, farblosen, störungsfreien Filmen auf, die auf dem Substrat sehr fest haften.

Die wäßrigen Lösungen sind deshalb vor allem für Lackierungen und Beschichtungen des unterschiedlichsten Substrate, z.B. von Metall, Glas, Holz, Textil und Leder geeignet und können mit beliebigen üblichen Hilfsmitteln verarbeitet werden. Sie können auf Grund ihrer hervorragenden Haftungseigenschaften auch für Verklebungen und Abdichtungen genutzt werden.

Eine bevorzugte Anwendung ist z.B. die Automobillackierung, wobei die erfindungsgemäßen wäßrigen Zubereitungen bevorzugt als klarer Decklack Verwendung finden.

### Beispiele

### Beispiel 1

### Herstellung von 1,4-Bis-(1,3,4-oxadiazolin-5-on-2-yl)-butan

In einer 4 l-Phosgenierapparatur wurde eine Lösung von 200 g Phosgen in 1,6 kg Phenol bei 20°C vorgelegt und mit 174 g Adipinsäuredihydrazid versetzt. Das Gemisch wurde unter Rühren innerhalb von einer Stunde auf 85°C erwärmt, wobei oberhalb einer Reaktionstemperatur von 45°C eine Gasententwicklung stattfand. Dann wurde bei 85°C unter Rühren 5 Stunden Phosgen in das Reaktionsgemisch eingeleitet (ca. 30 g pro Stunde). Anschließend wurde die Lösung 30 Minuten bei 85 - 90°C mit Stickstoff gespült und schließlich durch Vakuumdestillation bei 50 - 60°C vom größten Teil des Phenols befreit. Das zurückbleibende Produkt wurde bei 60°C in 1 l Toluol gelöst und durch Abkühlen auf 20°C ausgefällt. Der Niederschlag wurde abgesaugt, mit Toluol gewaschen und im Vakuum getrocknet.

Es wurden 188 g (83 % der Theorie) farblose Kristalle vom Schmelzpunkt 167 - 171°C erhalten. Nach dem Umkristallisieren aus Tetrahydrofuran besaß das Produkt einen Schmelzpunkt von 171 - 172°C.

Die Substanz ist in Wasser unlöslich.

### Beispiel 2

### Herstellung eines Polyamins

Man verfährt wie in Beispiel 1 der EP 0 392 299 A angegeben und erhält durch Formylierung eines Polyisocyanats und anschließende Überführung der resultierenden N-Formylgruppen in Aminogruppen ein farbloses Polyamin von 7,8 %. Das Polyamin ist sehr viskos bzw. in der Kälte wachsartig fest und für sich allein in Wasser nahezu unlöslich.

### Beispiel 3

### Herstellung einer erfindungsgemäßen Mischung

454,5 g des Polyamins aus Beispiel 2 werden bei ca. 60°C mit 453,5 g Wasser verrührt. Anschließend gibt man bei ca. 30°C 226 g der Substanz aus Beispiel 1 zu und rührt mit einem guten wirksamen Rührer bei hohen Scherkräften bis sich eine Lösung gebildet hat. Man gießt diese Lösung über ein Filter um geringe Trübungen zu entfernen. Man erhält eine klare, leicht gelbliche wäßrige Lösung mit einem Feststoffgehalt von ca. 60 %, die eine Viskosität von 250 mPas/23°C aufweist.

### Beispiel 4

Man verfährt wie in Beispiel 3 angegeben und stellt eine Mischung her aus
226 g Substanz aus Beispiel 1
359 g Polyamin aus Beispiel 2
und 586 g Wasser

Man erhält eine klare 50 %ige wäßrige Lösung mit einer Viskosität von 180 mPas/23°C.

### Beispiel 5

Man verfährt wie in Beispiel 3 angegeben und stellt eine Mischung her aus
226 g Substanz aus Beispiel 1
430 g Substanz aus Beispiel 2
23 g Hexamethylendiamin
679 g Wasser

Die ca. 50 %ige Mischung ist klar und hat eine Viskosität von 200 mPas/23°C.

### Beispiel 6

Die wäßrigen Muster aus den Beispielen 3 - 5 werden mit Hilfe einer 120 µ-Rakel auf saubere Glasplatten gestrichen und nach kurzem Ablüften bei 60°C, 80°C und 100°C in 30 min. ausgehärtet. Bei allen 3 Temperaturen entstehen völlig farblose Filme. Nach Einwirkung von Wasser, Wasser/Ethanol in 5 min. ist bei allen Filmen keine Veränderung zu beobachten. Mit einem Bleistift der Härte 5H läßt sich keine der Proben ritzen. Auch gegen Aceton zeigen die bei 80 und 100°C getrockneten Filme keine Veränderung.

Dieses Beispiel zeigt, daß aus den erfindungsgemäßen rein wäßrigen Lösungen flächige Kunststoffe mit einem sehr hohen Eigenschaftsniveau erhältlich sind.

## Patentansprüche

1. Mischungen aus Bis (1.3.4-Oxadiazolin-5-onen) der Formel (I) 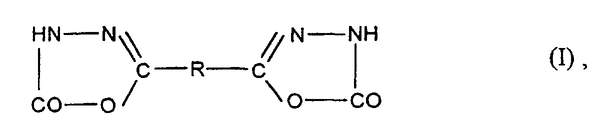 in der
R eine einfache Bindung oder einen bivalenten organischen Rest mit 1 bis 18 C-Atomen bedeutet, der aliphatisch, araliphatisch oder aromatisch sein kann,
mit organischen Polyaminen oder Polyaminabmischungen, die im Mittel mehr als 2 NH- und/oder NH₂-Gruppen enthalten.

2. Verwendung der Mischungen nach Anspruch 1 zur Lackierung von Substraten.

3. Verwendung nach Anspruch 2 als klarer Decklack.

4. Verwendung nach Anspruch 2 zur Automobillackierung.

## Claims

1. Mixtures of bis(1,3,4-oxadiazolin-5-ones) of the formula (I) 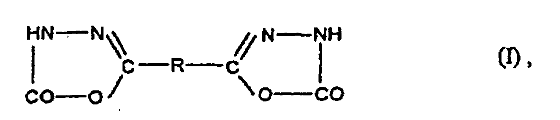 in which
R is a single bond or a divalent organic radical having 1 to 18 carbon atoms, it being possible for the said radical to be aliphatic, araliphatic or aromatic,
with organic polyamines or polyamine blends containing on average more than 2 NH and/or NH₂ groups.

2. Use of the mixtures according to Claim 1 for coating substrates.

3. Use according to Claim 2 as clear topcoat material.

4. Use according to Claim 2 for automotive finishing.

## Revendications

1. Mélanges de bis-(1,3,4-oxadiazoline-5-ones) de formule (I) 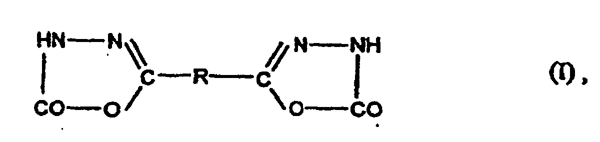 dans laquelle
R est une liaison simple ou un résidu organique bivalent avec 1 à 18 atomes C qui peut être aliphatique, araliphatique ou aromatique,
avec des polyamines organiques ou des mélanges de polyamines qui contiennent en moyenne plus de 2 radicaux NH et/ou NH2.

2. Mise en oeuvre des mélanges selon la revendication 1 pour la peinture de substrats.

3. Mise en oeuvre selon la revendication 2 comme couche de finition transparente.

4. Mise en oeuvre selon la revendication 2 comme peinture automobile.
